# EUROPEAN PATENT SPECIFICATION

(11) **EP 1 579 211 B1**
(45) Date of publication and mention of the grant of the patent: **21.02.2007**
(21) Application number: 02781710.5
(22) Date of filing: 23.12.2002
(51) Int. Cl.: G01N 33/53, G01N 33/543

(54) **MICROPOROUS FILTRATION BASED DOT IMMUNOASSAY DEVICE FOR METHOD FOR SCREENING OF ANALYTES AND METHOD OF USE**
MIKROPORÖSE FILTRATION-IMMUNOANALYSE-VORRICHTUNG UND VERFAHREN ZUR BESTIMMUNG VON ANALYTEN
DISPOSITIF DE DOSAGE IMMUNOLOGIQUE PAR POINT A FILTRATION MICROPOREUSE DESTINE A UN PROCEDE DE CRIBLAGE D'ANALYTES ET PROCEDE D'UTILISATION CORRESPONDANT

(43) Date of publication of application: 28.09.2005
(73) Proprietor: COUNCIL OF SCIENTIFIC AND INDUSTRIAL RESEARCH, New Delhi 110 001 (IN)
(72) Inventor: SURI, Chander, Raman, Chandigarh (IN); RAJE, Manoj, Chandigarh (IN); VARSHNEY, Grish, Chandra, Chandigarh (IN)
(74) Representative: Ahner, Francis
(86) International application number: PCT/IB2002/005607
(87) International publication number: WO 2004/057332

(56) References cited:
- EP-A- 0 439 017
- US-A- 4 675 299
- US-A- 4 913 791
- US-A- 5 503 741
- IJSSELMUIDEN, O.E.: "Optimising the solid-phase immunofiltration assay" JOURNAL OF IMMUNOLOGICAL METHODS, vol. 119, no. 6, 1989, pages 35-43, XP009010519 cited in the application

## Description

### Field of the invention

The present invention relates to an efficient and improved device for the screening of analytes using a microporous filtration based dot immunoassay method. More particularly this invention is based on the rapid solvent displacement method using a microporous pad. The invention is applicable for rapid screening of samples such as pesticides, hormones, and proteins with the ability of testing multiple samples in a single run. The device can be used for testing in all types of field conditions as it is extremely handy, versatile, efficient, self contained, ands meets with the prime objective of this invention to develop an improved dot blot method for rapid immunoscreening of substances in samples with the ability of testing multiple samples in a single run.

### Background of the invention

In prior art immunofiltration system developed by Pierce Corporation, USA wherein its Easy-Titer Enzyme linked immunofiltration assay kit (Immunofiltration Apparatus, Pierce catalogue, page: O-186) the solvent displacement is achieved by electrically operated vacuum pump. This makes it non-usable in remote locations having no access to electricity.

### Objects of the invention

The main objective of the present invention is to have a device for screening of analytes using a microporous filtration based dot immunoassay method.

Another objective of the present invention is to make this device efficient, effective, handy and operable in all kinds of field conditions.

### Summary of the invention

The principle of the present invention is based on rapid displacement of solvents under mild vacuum in solution-solid phase reaction of immunocomplex [Ijsselmuiden et al., J. Immunol. Methods, 6 (1989): 35]. In the present invention, this is achieved by a microporous absorbing pad upon which a nitrocellulose transfer membrane is placed. The absorbing pad under mild vacuum generated/regulated by running tap effectively filters out the unbound ligand and rinsing solutions through transfer membrane, thus enhancing the reaction kinetics of immunocomplex. This mechanism, in turn reduces the incubation steps of antibody-antigen reaction from hours to few minutes, allowing total assay time in less than 20 minutes.

Accordingly the present invention provides a microporous filtration based dot immunoassay device for screening of analytes, said device comprising three independent housings comprising of two upper housings and one lower housing, a transfer membrane, a microporous pad sandwiched between two upper housings, a polymer mesh placed over the lower housing, a seal means.

In one embodiment of the invention, the three housings comprise of one piece acrylic polymer sheet.

In another embodiment of the invention, wherein the transfer membrane is a nitrocellulose sheet.

In another embodiment of the invention, the absorbing pad is a high density polymeric sponge.

In another embodiment of the invention, the two upper housings have 32 holes each corresponding exactly to each other and all three housings are connected firmly through external connecting means.

In another embodiment of the invention, the seal means is an O ring.

In another embodiment of the invention, the analyte comprises of small molecules selected from small molecules of pesticides, proteins and hormones.

The present invention also relates to a method for microporous filtration dot immunoassay using a device comprising comprising three independent housings comprising of two upper housings and one lower housing, a transfer membrane, a microporous pad sandwiched between two upper housings, a polymer mesh placed over the lower housing, a seal means, said method comprising immobilizing a complimentary ligand to the analyte sample on the polymer mesh, adding a tracer along with the sample to each well, and then adding a substrate thereto, the enzyme tracer bound to membrane being converted to a colored product by a substrate and thereby functioning as an indicator of the proportion of the micromolecule in the sample.

In one embodiment of the invention, the color reaction of the tracer is stopped by means of a vacuum.

In another embodiment of the invention, the vacuum is generated through a running water tap.

In another embodiment of the invention, the analyte comprises small molecules selected from pesticides, hormones and proteins.

Accordingly, the present invention relates to a device for the screening of molecules using a microporous filtration based dot immunoassay method; such device being capable to screen multiple samples in a single run.

### Brief description of the accompanying drawings

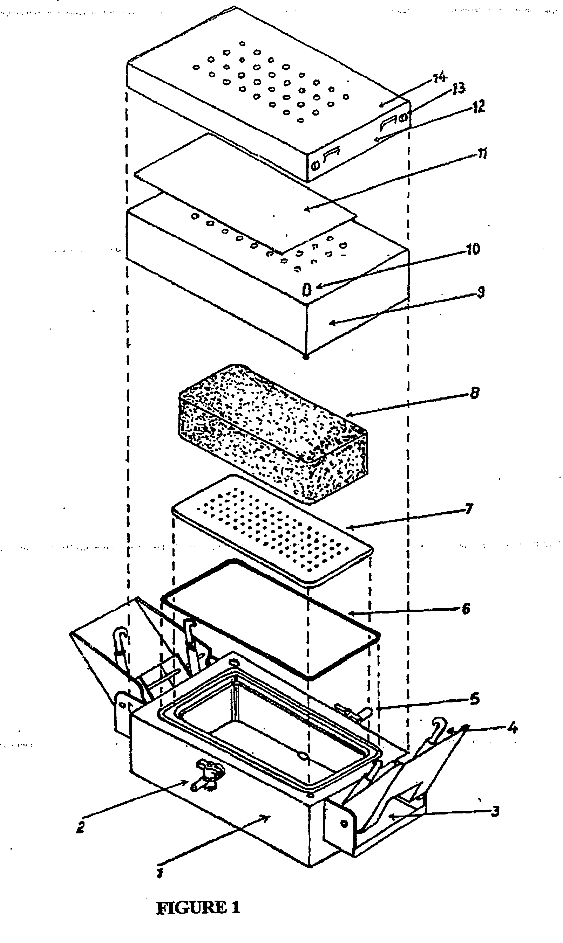

Figure 1 is a schematic representation of the device of the invention.

### Detailed description of the invention

The features and advantages of the invention will be more apparent from the following more particular description of the device, as illustrated in the accompanying drawing. The drawing is not necessarily to the scale, emphasis instead being placed upon illustrating the principles of the invention.

In this screening device the nitrocellulose membrane contains the bound complimentary ligand (anti-pesticide antibody) immobilized on it. The sample containing pesticide competes with an enzyme bound pesticide derivative (tracer) for the available antibody binding sites. The more the pesticide concentration in a sample, the less enzyme tracer available for antibody binding sites. The enzyme tracer bound to membrane is converted to a colored product by a substrate. The developed color is an indicator, which is inversely proportional to pesticide concentration in the sample.

The screening device consists of three separate housings (1, 9 and 12) each made of one-piece acrylic polymer (Perspex) sheet as shown in Figure 1. The transfer membrane (11) placed over the microporous pad (8) is sandwiched between the upper and the middle housings (9 and 12). The microporous pad is supported on a perspex mesh (7), which is placed over the lower housing (1) sealed via a silicon ring (6). The upper housing (12) contains 32 holes with silicon O-rings at bottom for blocking sample leakage. All three housings are held together via guided pins (10) and spring-loaded clamps (3, 4). The vacuum generated and regulated from running tap is connected to main system via vacuum line (2).

The other advantage of this device, unlike the existing method, is effective displacement of unbound ligand uniformly throughout the membrane surface by putting a microporous absorbing pad under the transfer membrane. The process of the present invention, hereby makes it very handy, portable, and does not require any power connection. It generates vacuum through running water, and only a few reagents are required for color development, and therefore most appropriate for every possible field application.

A further description of the invention is given in example below, which should not however be construed to limit the scope of the present invention unless otherwise stated.

### Example 1: Screening of smaller pesticide molecules (2,4-dichlorophenoxyacetic acid

The nitrocellulose transfer membrane placed between upper two Perspex housings is spotted with 10 µl of anti-2, 4-D antibody solution made in phosphate buffer saline (150 mM, pH 7.4)) in each well. The membrane is kept for incubation for 5 minutes, and dried under the mild vacuum (~ 0.25 kg/cm²). 100 µl PBS containing 0.5% Tween 20 (PBST) is then added to each well under same vacuum for removing unbound antibody molecules from membrane. The different concentrations of pesticide sample (1 ppb to 1 ppm) mixed with the enzyme tracer (pesticide-HRP) are added to each well and rinsed under mild vacuum. After rinsing again with PBST; the substrate (tetramethylbenzidine/H₂O₂) is added into each well to generate the color. The color reaction is stopped with water, which is then rinsed under mild vacuum. The intensity of color developed, inversely proportional to pesticide concentration in samples, is semi-quantified by comparing with standard test strip.

### Example 2: Screening of protein molecules, serum albumin in samples

The nitrocellulose transfer membrane is spotted with 10 µl of anti-BSA (bovine serum albumin) antibody solution made in phosphate buffer saline (PBS) in each well, kept for incubation for 5 minutes, and dried under the mild vacuum (~ 0.25 kg/cm²). 100 µl PBS containing 0.5% Tween 20 (PBST) is then added to each well under same vacuum for removing unbound antibody molecules from membrane. The different concentrations of BSA (1 ng/ml to 1 µg/ml) made in phosphate buffer are added to each well and rinsed under mild vacuum. After rinsing with PBST, the second antibody (goat-anti-BSA) labelled with HRP (1:20,000 dilution) was added into each well. After rinsing again with PBST, substrate (tetramethylbenzidine/H₂O₂) is added into each well. The color reaction is stopped with water, which is then rinsed under mild vacuum. The intensity of color developed, directly proportional to pesticide concentration is semi-quantified with a standard test strip.

## Claims

1. A microporous filtration based dot immunoassay device for screening of analytes, said device comprising three independent housings comprising of two upper housings and one lower housing, a transfer membrane placed over the microporous pad and sandwiched between two upper housings, a polymer mesh placed over the lower housing, a seal means.

2. A device as claimed in claim 1 wherein the three housings comprise of one piece acrylic polymer sheet.

3. A device as claimed in claim 1 wherein the transfer membrane is a nitrocellulose sheet.

4. A device as claimed in claim 1 wherein the microporous absorbing pad is a high density polymeric sponge.

5. A device as claimed in claim 1 wherein the two upper housings have 32 holes each corresponding exactly to each other and all three housings are connected firmly through external connecting means.

6. A device as claimed in claim 1 wherein the seal means is an O ring.

7. A device as claimed in claim 1 wherein the analyte comprises of small molecules selected from pesticides, proteins and hormones.

8. A method for microporous filtration dot immunoassay using a device comprising comprising three independent housings comprising of two upper housings and one lower housing, a transfer membrane placed over the microporous pad and sandwiched between two upper housings, a polymer mesh placed over the lower housing, a seal means, said method comprising immobilizing a complimentary ligand to the analyte sample on the polymer mesh, adding a tracer along with the sample to each well, and then adding a substrate thereto, the enzyme tracer bound to membrane being converted to a colored product by a substrate and thereby functioning as an indicator of the proportion of the micromolecule in the sample.

9. A method as claimed in claim 8 wherein the color reaction of the tracer is stopped by means of a vacuum.

10. A method as claimed in claim 8 wherein the vacuum is generated through a running water tap.

11. A method as claimed in claim 8 wherein the analyte comprises small molecules selected from pesticides, hormones and proteins.

## Patentansprüche

1. Auf mikroporöser Filtration basierende Dot-Immunoassayvorrichtung zum Screenen von Analyten, wobei die Vorrichtung drei unabhängige Gehäuse, bestehend aus zwei oberen Gehäusen und einem unteren Gehäuse, eine Transfermembran, welche über dem mikroporösen Kissen platziert und zwischen zwei oberen Gehäusen zwischengelegt angeordnet ist, ein Polymernetz, welches über dem unteren Gehäuse platziert ist, ein Abdichtungsmittel umfasst.

2. Vorrichtung nach Anspruch 1, wobei die drei Gehäuse aus einer aus einem Stück bestehenden Lage von acrylischem Polymer bestehen.

3. Vorrichtung nach Anspruch 1, wobei die Transfermembran eine Nitrocellulose-Lage ist.

4. Vorrichtung nach Anspruch 1, wobei das mikroporöse absorbierende Kissen ein offenzelliger Polymerschaumstoff mit hoher Dichte ist.

5. Vorrichtung nach Anspruch 1, wobei die beiden oberen Gehäuse 32 Löcher, die jeweils exakt einander entsprechen, aufweisen und alle drei Gehäuse durch äußere Verbindungsmittel fest miteinander verbunden sind.

6. Vorrichtung nach Anspruch 1, wobei das Abdichtungsmittel ein O-Ring ist.

7. Vorrichtung nach Anspruch 1, wobei der Analyt aus kleinen Molekülen, ausgewählt aus Pestiziden, Proteinen und Hormonen, besteht.

8. Verfahren für einen mikroporöse Filtration-Dot-Immunoassay unter Verwendung einer Vorrichtung, umfassend drei unabhängige Gehäuse, bestehend aus zwei oberen Gehäusen und einem unteren Gehäuse, eine Transfermembran, welche über dem mikroporösen Kissen platziert und zwischen zwei oberen Gehäusen zwischengelegt angeordnet ist, ein Polymernetz, welches über dem unteren Gehäuse platziert ist, ein Abdichtungsmittel, wobei das Verfahren umfasst, einen zu der Analyt-Probe komplementären Liganden auf dem Polymernetz zu immobilisieren, einen Tracer (Indikatorsubstanz) zusammen mit der Probe zu jeder Vertiefung hinzuzugeben und dann ein Substrat dazu hinzuzugeben, wobei der membrangebundene Enzym-Tracer durch ein Substrat in ein gefärbtes Produkt umgewandelt wird und **dadurch** als ein Indikator für den prozentualen Anteil des Mikromoleküls in der Probe funktioniert.

9. Verfahren nach Anspruch 8, wobei die Farbreaktion des Tracers mittels eines Vakuums gestoppt wird.

10. Verfahren nach Anspruch 8, wobei das Vakuum durch einen laufenden Wasserhahn erzeugt wird.

11. Verfahren nach Anspruch 8, wobei der Analyt kleine Moleküle, welche aus Pestiziden, Hormonen und Proteinen ausgewählt werden, umfasst.

## Revendications

1. Dispositif de dosage immunologique par points à base de filtration microporeuse permettant d'analyser des analytes, ledit dispositif comprenant trois logements indépendants constitués de deux logements supérieurs et d'un logement inférieur, une membrane de transfert placée par-dessus le coussinet microporeux et intercalée entre deux logements supérieurs, un filet en polymère placé par-dessus le logement inférieur, un dispositif de fermeture étanche.

2. Dispositif selon la revendication 1 dans lequel les trois logements sont constitués d'une feuille de polymère acrylique en un seul morceau.

3. Dispositif selon la revendication 1 dans lequel la membrane de transfert est une feuille de nitrocellulose.

4. Dispositif selon la revendication 1 dans lequel le coussinet absorbant microporeux est une éponge polymère de haute densité.

5. Dispositif selon la revendication 1 dans lequel les deux logements supérieurs possèdent 32 trous, correspondant exactement les uns aux autres, et les trois logements sont raccordés fermement par des moyens de raccordement externes.

6. Dispositif selon la revendication 1 dans lequel le dispositif de fermeture étanche est un joint torique.

7. Dispositif selon la revendication 1 dans lequel l'analyte est constitué de petites molécules choisies parmi les pesticides, les protéines et les hormones.

8. Procédé de dosage immunologique par points à base de filtration microporeuse faisant appel à un dispositif comprenant trois logements indépendants constitués de deux logements supérieurs et d'un logement inférieur, une membrane de transfert placée par-dessus le coussinet microporeux et intercalée entre deux logements supérieurs, un filet en polymère placé par-dessus le logement inférieur, un dispositif de fermeture étanche, ledit procédé comprenant le fait d'immobiliser un ligand complémentaire de l'échantillon d'analyte sur le filet en polymère, d'ajouter un marqueur en même temps que l'échantillon à chaque puits, puis d'y ajouter un substrat, le marqueur enzymatique lié à la membrane étant transformé en produit coloré par un substrat et jouant ainsi le rôle d'indicateur de la proportion de micromolécule dans l'échantillon.

9. Procédé selon la revendication 8 dans lequel la réaction colorée du marqueur est stoppée au moyen d'un vide.

10. Procédé selon la revendication 8 dans lequel le vide est créé par un robinet d'eau courante.

11. Procédé selon la revendication 8 dans lequel l'analyte comprend de petites molécules choisies parmi les pesticides, les hormones et les protéines.
